# EUROPEAN PATENT SPECIFICATION

(11) **EP 0 432 447 B1**
(45) Date of publication and mention of the grant of the patent: **07.09.1994**
(21) Application number: 90121229.0
(22) Date of filing: 06.11.1990
(51) Int. Cl.: C07C 2/12, C07C 2/00

(54) **Process for upgrading light olefinic streams**
Verfahren zur Qualitätserhöhung von Leichtolefinströmen
Procédé pour l'amélioration de qualité de courants d'oléfines inférieures

(30) Priority: 16.11.1989 US 437137; 16.11.1989 US 437138
(43) Date of publication of application: 19.06.1991
(73) Proprietor: MOBIL OIL CORPORATION, Fairfax, Virginia 22037-0001 (US)
(72) Inventor: Beech, James Harding, Jr., New Castle DE 19810 (US); Ragonese, Francis Paul, County of Mercer, NJ 08512 (US); Stoos, James Arthur, Camden NJ 08012 (US); Yurchak, Sergei, Delaware, PA 19063 (US)
(74) Representative: Kador & Partner

(56) References cited:
- DD-A- 273 055
- US-A- 4 528 414
- US-A- 4 544 792
- US-A- 4 717 782
- "Zeolite Chemistry and Catalysis", Rabo, ACS, Washington DC (1976), pages 714, 482 and 487

## Description

The invention relates to upgrading of light olefinic streams, ranging from ethylene containing streams up to naphtha boiling range materials, to produce high quality distillate and motor fuel.

Upgrading of light olefinic streams by oligomerization over acid-type catalysts has long been known. Solid phosphoric acid catalysts, or liquid phosphoric acid on a support, have been used to polymerize propylene and butenes to gasoline boiling range materials. The acid required a certain amount of water, so contacting the feed with water was a convenient way to add water, and also to remove nitrogen compounds which were catalyst poisons. The nitrogen content of the feed had to be limited to less than 50 ppm basic nitrogen for satisfactory life of the phosphoric acid catalyst.

An extensive review of more modern oligomerization technology is reported in Conversion of C₂-C₁₀ to Higher Olefins over Synthetic Zeolite ZSM-5, ACS Symposium Series, No. 218, Intrazeolite Chemistry (1983).

US-A-4,544,792 discloses use or moderate temperature and relatively high pressure in olefin oligomerization to favor aliphatic, distillate range product.

In the upgrading of Fischer-Tropsch olefins it has been found beneficial to water wash a C₃-C₇ olefinic stream to reduce the oxygenate content to below 1 wt%, followed by oligomerization in a pressurized reactor in the presence of hydrogen. US-A-4,554,792 discloses that feedstocks containing trace amounts of oxygenates can be catalytically upgraded over H-ZSM-5 when as little as 1 mole % H₂ is present. At increased oxygenate concentrations more hydrogen is required. The cofed hydrogen inhibits the formation of coke on the catalyst.

US-A-4,513,156 discloses removal of essentially all the oxygenates of a Fischer-Tropsch liquid upstream of the olefin oligomerization reactor, using a multi-stage vertical extraction column for water washing or extracting oxygenates.

Upgrading of pyrolysis naphtha produced during steam cracking to make ethylene, by passing the naphtha over Pd/Zn/ZSM-5 at 900 to 1200°F, is disclosed in US-A-4,097,367.

Refiners have been reluctant to subject olefinic streams to hydrotreating, or to catalytic hydroprocessing, because of the risk that the olefins would be saturated. In gasoline boiling range materials this saturation would greatly decrease the gasoline octane. In C₂ to C₄ olefins, saturation of the olefins would reduce greatly their reactivity, and reduce their value. C₂ olefins are very difficult to oligomerize, and C₂ paraffins are even less reactive. The presence of large amounts of basic nitrogen in these streams makes hydrotreating desirable, but the need to preserve olefins makes it unwise.

Feed contamination is a severe problem with refinery olefinic streams because these streams usually contain large amounts of sulfur and nitrogen. Much of the nitrogen contamination can be introduced in trying to reduce the sulfur contamination, since amine scrubbing removes sulfur but leaves trace amounts of amine in the scrubbed stream.

The presence of basic nitrogen in light olefin refinery streams can damage zeolite oligomerization catalysts, so it would be beneficial to render such catalysts more tolerant of such catalyst poisons. A feed pretreatment process which could reduce the level of nitrogen contaminants in the feed at least two orders of magnitude below the 50 ppm tolerated by the phosphoric acid olefin oligomerization process, and also tolerate momentary overloads and upsets, would be valuable.

Such a pretreatment process should be capable of operating continuously, or at least for long periods using a regenerable, rather than replaceable, nitrogen removal medium. Feed pretreatment can be further complicated by the presence of large amounts of unusual contaminants in refinery light olefin streams, especially of dienes, which can irreversibly deactivate some nitrogen removing materials. Thus a conventional nitrogen sorbent which is regenerable in some other service might not prove suitable for use in feed pretreatment of light olefinic streams prepared by high temperature thermal and/or catalytic processing of crude oil.

Thus poor quality or hard-to-process gasoline boiling range streams such as coker, visbreaker or pyrolysis naphtha, have long been a problem for refiners. These materials contain such high quantities of di-olefins, in addition to sulfur and nitrogen compounds, that they are extremely difficult to process in conventional refinery units. The large di-olefin content of such streams renders them extremely reactive or unstable. If an attempt is made to simply hydrotreat these streams in a conventional hydrotreater, the reactive di-olefins form gum which plugs the conventional hydrotreating bed, or less frequently plugs the heat exchanger or heater upstream of the hydrotreating unit. These unstable naphthas are of such poor quality that they cannot be blended into the refinery gasoline pool, and refiners have resorted to extreme measures in order to deal with them.

Another problem with light olefinic streams from a refinery, besides the difficulty in pretreating such streams, is that refinery propylene frequently contains large amounts of ethylene. There is a need for an upgrading process which will efficiently convert ethylene as well as heavier olefins, desirably one capable of operating for a long time at relatively high severity and at relatively high space velocities.

We have discovered an efficient light olefin upgrading process, which can tolerate the presence of large amounts of ethylene in a propylene rich stream, and which will efficiently convert a mixture of light olefins to heavier, more valuable normally liquid products. The process tolerates the presence of relatively large amounts of dienes.

We have also discovered a feed pretreatment process which provides an ideal way to efficiently remove basic nitrogen contaminants from refinery light olefin streams and make these streams suitable for downstream zeolite upgrading.

Accordingly, the present invention provides a process for upgrading a C₂-C₁₀ olefinic feed containing basic nitrogen compounds, produced by thermal or catalytic cracking of a heavier liquid hydrocarbon and selected from coker naphtha, visbreaker naphtha, pyrolysis naphtha, olefinic naphtha from cracking, refinery-produced LPG streams comprising 2-5 carbon atom hydrocarbons and normally gaseous 3-4 carbon atom hydrocarbon streams produced by cracking, said process comprising contacting the olefinic feed with a catalyst comprising a zeolite having a Constraint Index of 1 to 12 and a silica to alumina mole ratio of at least 12 at superatmospheric pressure and in the presence of hydrogen at a hydrogen to hydrocarbon ratio of about 0.01:1 to 1:1 to oligomerize at least a portion of the olefins in the feed to gasoline and distillate boiling range products, and wherein the presence of hydrogen extends the catalyst life of the upgrading catalyst relative to operation at superatmospheric pressure in the absence of added hydrogen.

In another embodiment, the present invention provides a process for oligomerizing a feed of a light, normally gaseous hydrocarbon stream containing more than 0.5 wt ppm basic nitrogen compounds and comprising olefinic hydrocarbons having three to four carbon atoms produced by catalytic cracking of heavy, sulfur containing hydrocarbons boiling in the gas oil and heavier range to lighter products and H₂S, which lighter products are treated with an amine to reduce the H₂S content and produce a light, normally gaseous hydrocarbon stream containing a reduced H₂S content and more than 0.5 wt ppm basic nitrogen as a result of the amine treatment of the lighter products, to produce stable gasoline and distillate boiling range products comprising reducing the basic nitrogen content of the light, normally gaseous hydrocarbon feed by contacting said feed with a sorbent having an affinity for basic nitrogen compounds to reduce the basic nitrogen content of the feed below 0.1 wt ppm; and oligomerizing said feed with a basic nitrogen content below 0.1 wt ppm over an oligomerization catalyst comprising a zeolite having a Constraint Index of about 1 to 12 and a silica to alumina mole ratio of at least 12 at olefin oligomerization conditions to produce a gasoline and distillate boiling range product.

In a more limited embodiment, the present invention provides a process for producing gasoline and distillate boiling range products from heavy hydrocarbon feed boiling in the gas oil and heavier range comprising catalytically cracking said heavy feed at catalytic cracking conditions in a catalytic cracking unit to produce cracked products including olefinic C₃ and C₄ hydrocarbons and H₂S; treating the olefinic C₃ and C₄ hydrocarbons with an amine to reduce the H₂S content thereof and produce treated olefinic C₃ and C₄ hydrocarbons containing more than 0.1 wt ppm basic nitrogen compounds as a result of the amine treating step; reducing the basic nitrogen content of the olefinic feed below 0.05 wt ppm basic nitrogen by extracting the basic nitrogen compounds from the feed with water to produce a water extracted olefinic feed with a reduced basic nitrogen content; and oligomerizing the water extracted olefinic feed by contact with an upgrading catalyst comprising ZSM-5 having a silica to alumina mole ratio of at least 12, and an alpha value, on a pure ZSM-5 basis, or at least 100, at olefin oligomerization conditions including a temperature of 177 to 399°C (350-750°F), the upgrading reaction is conducted in the presence of hydrogen, at a mole ratio of hydrogen to hydrocarbon of about 0.01:1 to about 0.5:1 and a hydrocarbon weight hourly space velocity of about 0.1 to 10 to oligomerize at least a portion of the olefins in the feed to a gasoline and distillate boiling range product.

In another embodiment, the present invention provides a process for upgrading an unstable coker naphtha containing olefins and more than 0.25 wt% dienes and more than 1.0 wt ppm basic nitrogen compounds to stable gasoline and distillate boiling range products comprising reducing the basic nitrogen content of the feed by contacting the feed with a material selected from the group of water and acidic solids to reduce the basic nitrogen content below 0.1 wt ppm; and upgrading the resulting unstable coker naphtha with a reduced basic nitrogen content by contact with an upgrading catalyst comprising a zeolite having a Constraint Index of about 1 to 12, a silica to alumina mole ratio of at least 12, and an alpha value, on a pure zeolite basis, or at least 100, at olefin oligomerization conditions including a temperature, hydrocarbon feed space velocity, hydrogen/hydrocarbon ratio and catalyst alpha value sufficient to oligomerize at least a portion of the olefins in the feed to produce a gasoline and distillate boiling range product and catalytically converting at least a majority of the dienes in the coker naphtha to produce a stable gasoline and distillate product with a reduced diene content of less than 0.1 wt%.

In a more limited embodiment, the present invention provides a process for upgrading an unstable coker naphtha containing olefins and more than 0.25 wt% dienes and more than 1.0 wt ppm basic nitrogen compounds to stable gasoline and distillate boiling range products comprising reducing the basic nitrogen content of the feed below 0.05 wt ppm basic nitrogen by contacting the feed with a material selected from the group of water and acidic solids to produce an unstable coker naphtha with a reduced basic nitrogen content; and upgrading the resulting unstable coker naphtha with a reduced basic nitrogen content by contacting the naphtha with an upgrading catalyst comprising ZSM-5 having a silica to alumina mole ratio of at least 12, and an alpha value, on a pure ZSM-5 basis, or at least 100, at olefin oligomerization conditions including a temperature of 121 to 232°C, a coker naphtha space velocity of about 0.1 to 10 and a hydrogen/hydrocarbon mole ratio of 0.5 to 5 to oligomerize at least a portion of the olefins in the feed to a gasoline and distillate boiling range product while catalytically converting at least a majority of the dienes in the coker naphtha to produce a stable gasoline and distillate product with a reduced diene content.

### BRIEF DESCRIPTION OF THE DRAWINGS

Figure 1 shows the distribution of DEA between water and LPG.

Figure 2 shows the effectiveness of several sorbents for removal of DEA from hexene.

Figure 3 shows basic nitrogen in oligomerized liquid product as a function of feed pretreatment and H₂ addition.

The feedstocks which are suitable for use in the present invention are any light olefinic fractions produced by thermal or catalytic cracking of crude oil fractions.

The preferred feedstocks are light olefin streams from a catalytic cracking unit. The process works especially well when the feed is an LPG fraction which has been treated with a basic nitrogen containing steam such as DEA, di ethanol amine, to remove sulfur compounds. The process tolerates very well C₂ to C₁₀ olefin containing streams containing in excess of 1 ppm basic nitrogen, though a feed pretreatment step is advantageous in reducing the basic nitrogen content.

An especially preferred feedstock is a liquified petroleum gas, LPG, containing 0.1 to 0.2 wt ppm basic nitrogen by conventional analytical methods, undetectable levels of sulfur, no more than 0.5 wt% dienes, less than 5 ppm ammonia, typically on the order of 2-4 ppm ammonia, no more than 1 ppm sodium and having a low content of other metals. Although such a feed might seem to be fairly clean for a commercially available LPG, it is difficult to treat using conventional olefin upgrading technology, and is therefore an ideal candidate for use in the practice of the present invention.

The process of the present invention can also be used for upgrading FCC naphtha, or coker naphtha or any naphtha obtained by a thermal or catalytic process.

Suitable naphtha feedstocks may be produced using the fluid-coking process described in US-A-2,905,629, 2,905,733 and 2,813,916. The Flexicoking process may also be used. This process is identical to fluid-coking but converts coke to low BTU gas. Flexicoking is described in US-A-3,661,543, 3,816,084, 4,055,484 and 4,497,705.

The naphtha boiling range streams may comprise C₄-C₁₂+ materials, and usually comprise C₅-C₁₂ materials Expressed as a boiling range, the naphtha will usually have an initial boiling point of 15.5 to 65.5°C or above, and an end boiling point in the range of 149 to 204.5°C. The process of the present invention tolerates even heavier charge stocks, those having an end point up to 232 to 260°C, but such materials are usually considered too heavy for use in a refinery gasoline pool and for that reason are not preferred feedstocks for use herein. They may be included in the feedstock to the process of the present invention, and the heavy ends removed from the product by distillation.

Unstable naphthas, such as coker naphtha, can be processed according to the invention, although their high diene content tends to cause polymerization at low temperatures. These materials generally have ratios of n-olefins to paraffins in excess of 1. In the C₆ to C₁₂ range, these ratios frequently range from about 1.1 to 2.1. The ratio usually increases with increasing carbon numbers. Extensive analysis of coker naphtha, and some of its characteristics, are reported in US-A-4,711,968.

Any zeolite having a silica/alumina mole ratio greater than 12 and a Constraint Index of 1-12 can be used as oligomerization catalyst. Details of the Constraint Index test procedures are provided in J. Catalysis 67, 218-222 (1981) and in US-A-4,711,710. Preferred zeolites are ZSM-5, ZSM-11, ZSM-12, ZSM-23, ZSM-35, ZSM-48 and ZSM-57, respectively defined by the x-ray data set forth in US-A-3,702,886, 3,709,979, 3,832,449, 4,076,842, 4,016,245, EP-A-151,132 and US-A-4,046,859.

Zeolites in which some other framework element is present in partial or total substitution of aluminum can be advantageous, as can be high-silica forms of ZSM-5 such as described in US-A-4,061,724 ("Silicalite"). Elements which can be substituted for part or all of the framework aluminum are boron, gallium, zirconium, titanium and other trivalent metals which are heavier than aluminum. Specific examples of such catalysts include ZSM-5 or zeolite beta containing boron, gallium, zirconium and/or titanium. In lieu of, or in addition to, being incorporated into the zeolite framework, these and other catalytically active elements can also be deposited upon the zeolite by any suitable procedure, e.g., impregnation. Preferably relatively high silica zeolites are used, with a silica/alumina ratio above 20/1, and more preferably with a ratio of 70/1, 100/1, 500/1 or even higher.

Advantageously the zeolite has relatively high acid cracking activity, or alpha activity. Preferably the alpha value of the pure zeolite is in excess of 100, and most preferably is about 150 to 250. The high acid activity allows the desired conversion reactions to be achieved at relatively low temperatures. Somewhat higher temperatures can be tolerated, but this can increase the rate of gum formation and increase catalyst deactivation rates. The Alpha Test is described in US-A-3,354,078 and in the Journal of Catalysis, Vol 4, p 527 (1965); Vol 6, p 278 (1966); and Vol 61, p 395 (1980).

It is preferred to keep temperatures fairly low, particularly when there are large amount of dienes in the feed. The temperature, space velocity and catalyst alpha activity can all vary widely within the permitted limits, provided that dienes, when present, are catalytically converted at temperatures low enough to substantially eliminate diene reactions that are thermally induced. Typically at least 90% of diene conversion is catalytic, less than 10% thermal.

Suitable conditions are listed in the following table.

| | Acceptable | Preferred | Most preferred |
|---|---|---|---|
| Reactor Temp °C | 93-482 | 121-399 | 149-288 |
| Pressure, bar | any | 7.9-70 | 21.7-56 |
| WHSV (on olefin) | 0.1-100 | 0.2-20 | 0.5-5 |
| Delta T, °C | 83 | 55.5 | 27.7 |
| Inert Diluent,mole % | 0-95 | 0-90 | 20-60 |

When a fluidized bed reactor is used the delta T will be very low, or zero. The above table shows the maximum delta T preferred for fixed bed operation.

Particularly suitable reaction conditions for use with unstable naphtha feeds containing dienes and olefins produced by thermal cracking are listed in the following table.

| | Acceptable | Preferred | Most preferred |
|---|---|---|---|
| Reactor Temp °C | 93-371 | 107-260 | 121-232 |
| Pressure, bar | 1-111.4 | 7.9-70 | 21.7-35.5 |
| WHSV (on olefin) | 0.1-100 | 0.2-20 | 0.5-5 |
| Delta T, °C | 83 | 55.5 | 27.7 |
| H₂ or inert mole % | 0-95 | 0-90 | 20-60 |

The presence of hydrogen is beneficial in extending catalyst life and increasing distillate yields and conversion of reactants. The hydrogen probably does not react with oxygen compounds in the feed, as taught for instance in US-A-4,544,792. It is believed that the hydrogen does not act with any metal on the catalyst to hydrogenate olefins or dienes. The process works well when the catalyst has no hydrogenation/dehydrogenation components, and is in the H-form. The zeolite is believed, at the reaction conditions used herein, to create small amounts of atomic hydrogen which is extremely reactive and which reacts with the diene components of the feed, or reactive intermediates formed by the dienes and to a lesser extent with olefins in the feed. The hydrogen may also react with nitrogen impurities which are in the feed, and release ammonia, and this may in some way alter the selectivity of the catalyst. The hydrogen probably also hydrogenates, to some extent, coke precursors.

Some benefits will be seen when the feed contains as little as 1 to 5 mole % H₂. Operation with 5 to 50 mole % H₂ in the reaction zone is preferred, with 10 to 30 mole % H₂ being especially preferred. Operation with 0.1:1 to 1:1 H₂:hydrocarbon ratios (molar basis) gives good results. Pretreatment, when practised, should reduce the basic nitrogen content of the feed to less than 1 ppm, preferably to less than 0.5 wt ppm, and ideally to less than 0.1 wt pm basic nitrogen, and most preferably to 0.05 wt ppm basic nitrogen, or less.

Many refinery LPG streams will, using conventional methods of analysis such as UOP method 430-70T, appear to have relatively low levels of nitrogen (of the order of 0.1-0.2 ppm) while actually having about 1 ppm nitrogen. This test method involves flashing the vapor, passing the vapor through an acid wash, and analyzing the acid spectrophotometrically for nitrogen. It has been determined that this is not a reliable test method for assaying 0.05 to 0.1 ppm nitrogen in a refinery LPG stream in a refinery laboratory. A simple, and accurate superior analytical procedure comprises using multiple stages of water extraction to remove the nitrogen compounds from the LPG, and then analysing the water extract from each stage for nitrogen. Using this method the refinery LPG stream referred to above consistently had about 1 ppm nitrogen, whereas the flash vaporization method gave results of 0.1 to 0.2 ppm nitrogen. According to the vaporization procedure roughly equal weights of water and LPG are added to an LPG cylinder which is rolled on a drum roller for 0.5-1 hour, and then allowed to stand for about 0.5 hour for phase separation. The water phase is then drained out of the cylinder through the gas valve, using a high pressure flexible hose. Both the water extract and the LPG (via another water extraction on a small sample) are then analyzed for nitrogen.

Water washing, preferably with a slightly acidified water stream, can reduce the combined nitrogen content of the feed to the desired level.

Solid acidic substances may be used to pretreat the feed. Such acidic materials include ion exchange resins in the acid form, activated alumina, fresh or spent FCC catalyst, shape selective zeolites, and the like.

Process conditions for feed pretreatment, when using a solid bed, include the following:

| | Acceptable | Preferred | Most preferred |
|---|---|---|---|
| Temperature °C | 10-121 | 15.5-65.5 | 21.1-37.7 |
| Pressure | ----- liquid phase preferred ----- | | |
| WHSV | 0.01-100 | 0.1-50 | 0.5-20 |
| Maximum ppm N In Effluent | 1 | 0.1 | 0.05 |

The process works especially well when a combination of pretreatment processes is used, i.e., when there are at least two stages of feed pretreatment to remove nitrogen.

Liquid/liquid extraction of basic nitrogen compounds is a preferred pretreatment method. Any liquid which has an affinity for basic nitrogen compounds, and which is immiscible with the hydrocarbon feed, can be used. Suitable liquid extractants can be selected from water, and other liquids such as Sulfolane, glycols, alcohols, and mixtures thereof. Liquid/liquid extraction is preferred over use of acidic solids. Ion exchange resins will remove basic nitrogen from the feed, but cannot be regenerated.

Water washing, preferably with acidified water, is an especially preferred way to remove the bulk of the basic nitrogen compounds present. A simple, multi-stage water washing feed pretreatment is effective and can be run continuously. The only drawback to water washing is that its efficiency declines when the amine concentration in the hydrocarbon stream to be treated is unusually low, or unusually high.

The distribution coefficient for DEA between H₂O and LPG was determined experimentally by contacting DEA-containing water with pure Matheson LPG and multiple contacts of a refinery LPG stream with pure water.

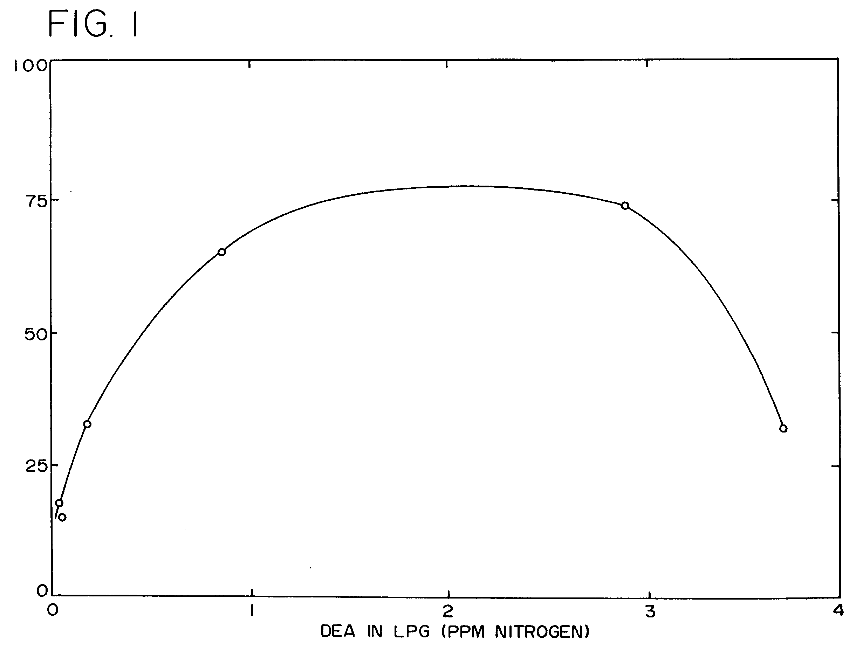

As shown in Figure 1, the distribution coefficient increased from about 15 for 0.05 ppm N in LPG to about 65 for 1 ppm N. At high levels of N, around 130 ppm in LPG, the distribution coefficient decreased to about 3. In the range of 2-5 ppm N, the water nitrogen levels are outside the test limits and no quantitative results were obtained: however qualitatively the distribution coefficient in this region is above 20.

Because it was difficult to measure accurately these low nitrogen levels in hydrocarbon, the N levels were calculated from the difference in the water N levels, so there is some uncertainty about the distribution coefficient at the 0.2 and 0.9 ppm N in LPG level.

A distribution coefficient no larger than, for example between 10 and 11, makes for effective design. Using a value of 11.5 for a distribution coefficient, 240 lb/min (28 gpm) of water is required to reduce 750 lb/min of LPG from 3 ppm N to 0.05 ppm N in a 3 stage countercurrent extraction column.

Where costs of liquid/liquid extraction to remove basic N must be minimized, or to achieve an added measure of security, one or more stages of solid extraction may be substituted for, or used in conjunction with, the liquid/liquid extraction step.

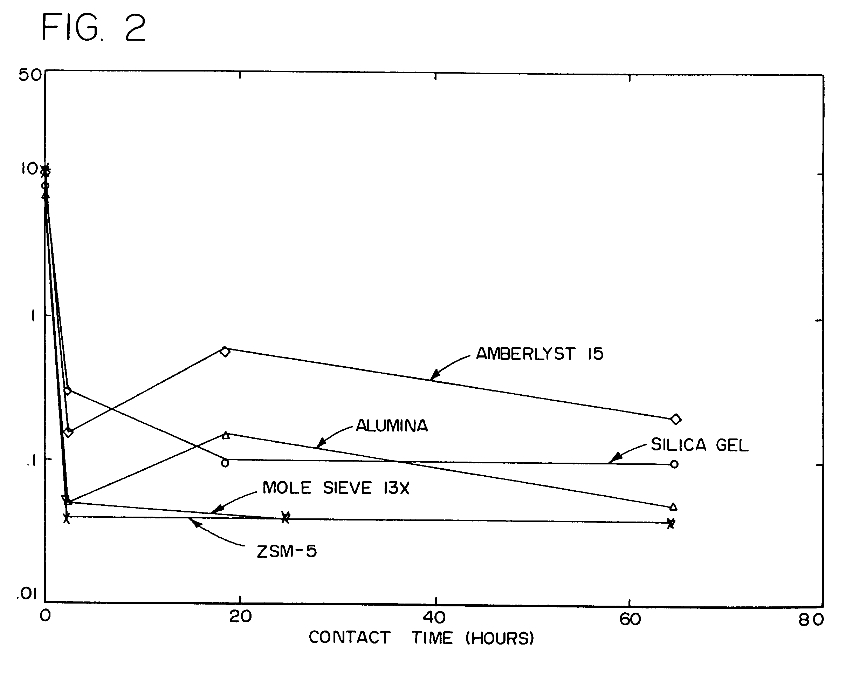

Figure 2 shows the relative efficiency of various sorbents for removal of basic N from hexene/DEA mixtures. Hexene was used, rather than LPG because hexene is much easier to work with in the laboratory, and the results are believed comparable. This Figure shows that various solid sorbents can also be used or hexene/DEA mixtures. When sorbent beds are used downstream of, or instead of, a liquid/liquid extraction zone, the beds are preferably sized to provide a liquid hourly space velocity of 0.1 to 10 hr⁻¹, and most preferably a liquid hourly space velocity of 0.2 to 5 hr⁻¹. Sorbent bed size should be increased about 2-3 fold when no water washing is used upstream. Sorbent beds in parallel may be used to permit on stream regeneration of an absorbent bed. Beds of ion-exchange resin may be regenerated by conventional means, e.g., by circulating an acid solution over the bed, but if polymers or tar are formed on the resin bed by catalytic action the resin will have to be replaced rather than regenerated.

Zeolitic materials, and other solids which can tolerate high temperatures, may be regenerated by heating to a high enough temperature to desorb the adsorbed basic nitrogen compounds. Alumina may be regenerated by air oxidation to oxidize or drive off the basic nitrogen containing compounds. Oxidative regeneration can be used to remove polymer or tar that forms on such sorbent beds via catalytic reactions.

When naphtha boiling range feeds are used, they may contain large amounts of dienes (rendering them unsuitable for conventional hydroprocessing) and large amounts of sulfur. The process of the present invention tolerates these poisons. When large diene contents are present, in excess of 0.5 wt%, then reaction temperatures in the olefin upgrading step should, for the initial stages at least, be kept relatively low to minimize gum formation in the preheater.

The process of the present invention works especially well when both hydrogen co-feed and feed pretreatment, to remove basic nitrogen compounds, are practised together. The combination permits unusually long run lengths to be achieved, or very high space velocity to be maintained. Although the reason for the unexpected increase in catalyst stability is not known, it is speculated that each step removes most efficiently the poisons left behind by the other step. The poisons not efficiently removed by water washing are the poisons most readily removed by the presence of hydrogen in the oligomerization reactor. The two steps are complementary, and make the process far more tolerant of unpredictable changes that are an everyday occurrence in modern refineries.

### EXPERIMENTS

Several experiments were conducted to test the effectiveness of various feed pretreatments and of hydrogen co-feed, using the same feed, a specified feed pretreatment (ranging from none to water washing to adsorbent bed contact) and the same oligomerization treatment. The feedstock analysed:

| | |
|---|---|
| C3= | 30.7 wt% |
| C3 | 10.7 wt% |
| 1,3 C4== | 0.4 wt% |
| T2 C4= | 9.0 wt% |
| C2 C4= | 6.1 wt% |
| iC4= | 14.1 wt% |
| 1 C4= | 8.8 wt% |
| iC4 | 15.3 wt% |
| nC4 | 4.6 wt% |
| | 100.0 |
| Basic Nitrogen 1.01 wt ppm | |

All feed pretreatment was carried out in the liquid phase, at 18.25 bar (250 psig). When a solid bed feed pretreater was used, the bed contained 275g of Amberlyst-15® resin. The feed rate was 70 cc/hr, downflow through the bed, for a weight hourly space velocity of 0.15. The material exiting the resin bed was estimated to contain slightly less than 0.1 wt ppm basic N. When water washing was used for feed pretreatment, the feed was passed through three stages of contact with equal volumes of water at room temperature. The basic nitrogen content of the water washed feed was estimated to be below 0.1 wt ppm.

A longer term test was conducted using Amberlyst 15 (Trademark) ion exchange resin to pretreat an LPG feed. This resin was chosen because of its ready availability commercially, and because it had previously been used successfully to remove nitrogen compounds from jet fuel.

A 1000 cc hoke vessel was filled with 275g of fresh dry resin in the H+ state. The resin was preswelled with hexene and used on line to treat a refinery LPG stream at a 0.15/hour space velocity. Calculations indicated that this amount of Amberlyst 15 should have lasted more than 50 years, based on 1 ppm N in the feed, assuming the acid sites on the resin were used stoichiometrically. While the unit worked efficiently at first to remove nitrogen, and did improve the operation of a downstream olefin oligomerization unit, there was an apparent nitrogen breakthrough after only 14 days on stream. The breakthrough was confirmed by the measured reduction in acid sites on the resin from 3.5-3.8 meq/gm to 1.8 meq/gm, uniformly through the resin bed. Attempts at regenerating the resin by ion exchange with acid resulted in an increase in acid sites to 2.5 meq/gm, indicating that the resin acid sites had become permanently blocked.

An analysis of spent and fresh resins was conducted to try to determine what had caused the permanent deactivation. The spent resin contained a higher percentage of C-H containing species than the fresh resin. It is believed that the deactivation of the Amberlyst resin resulted from polymerization of one of the feed components which resulted in pore plugging.

Because of the catalytic activity of some sorbents, such as the ion exchange resins, and the reactivity of the feeds contemplated for use herein, it usually will be preferred to operate with water extraction rather than a solid sorbent bed. If a sorbent bed is to be used, it should first be tested in the laboratory to determine that it is efficient at absorbing nitrogen compounds, and free of undesirable catalytic properties.

All tests were run at 56.2 bar, (800 psig) in the oligomerization reaction zone. The oligomerization reactor was a fixed bed reactor, having an internal diameter of 1.25 cm and containing 60 cc of catalyst in each of 3 beds in series. When hydrogen was added to the feed, it was added at a rate of 44.5 m³/m³ (250 SCFB).

The olefin oligomerization catalyst was a 65/35 mix of H-ZSM-5 in alumina. The ZSM-5 had a silica to alumina mole ratio of 70/1 and an alpha activity (pure zeolite basis) of 230-240. Each test lasted about three weeks. Reaction conditions included a temperature of 249-304.5°C (480-580°F) and a 1.0 WHSV on olefin.

The total liquid product from the oligomerization reactor was analyzed for basic nitrogen. This is an indication whether or not the catalyst has been poisoned with basic nitrogen compounds. Because the catalyst acid sites will react with, or complex, basic N, the presence of basic N in the liquid product is an indication that the catalyst bed is saturated with, or poisoned by, basic N entering with the feed.

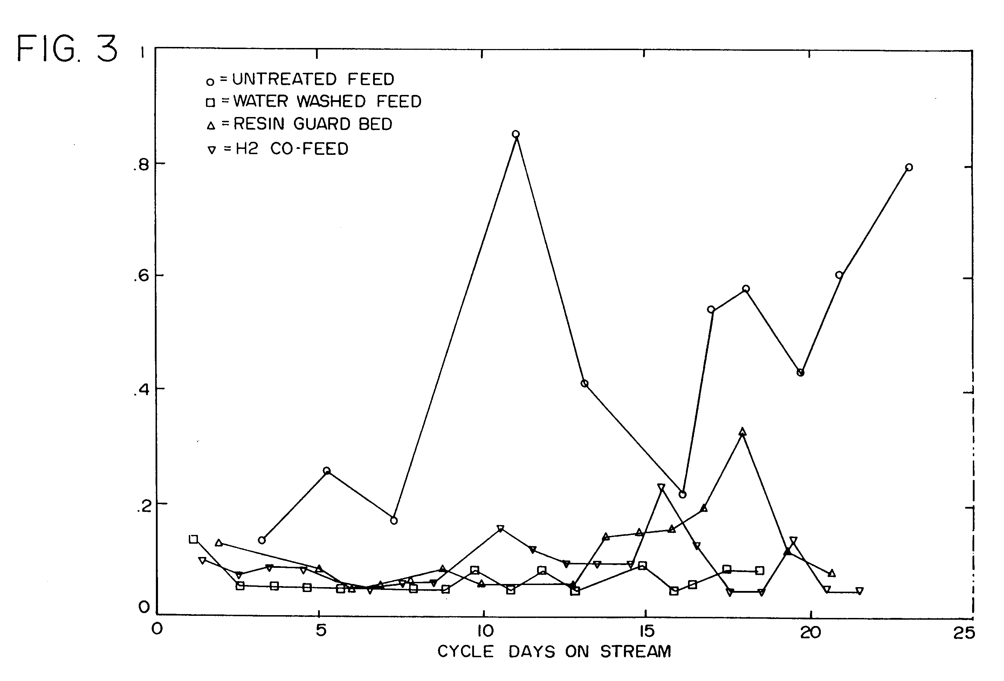

The experimental results are resented graphically in Figure 3, where:
○ = Untreated Feed (1.0 ppm N)
□ = Water Washed Feed (less than 0.1 ppm N)
△ = Resin Guard Bed (about 0.1 ppm N)
∇ = H₂ co-feed (the feed contained 1.0 ppm N)
Untreated feed rapidly deactivates the catalyst. The data in Figure 3 show that there is rapid contamination of the bed of oligomerization catalyst when untreated feed is used. This means that the oligomerization reactor performance will decline rapidly, and/or that the oligomerization reactor must be frequently regenerated.

Water washing of feed is effective at preventing nitrogen contamination of the oligomerization catalyst. Resin guard bed treating is effective for a time, but in the example there was eventually breakthrough of basic nitrogen into the product, indicating some poisoning of the oligomerization catalyst. Hydrogen co-feed was very effective at eliminating basic nitrogen compounds from the oligomerization zone liquid product.

Preferably, feed pretreatment to reduce the total nitrogen content is coupled with hydrogen addition to the oligomerization reactor to achieve best results. To some extent, better feed preparation will compensate for lower hydrogen partial pressure, and vice versa. Ideally, essentially all of the basic nitrogen in the feed is removed by the feed pretreatment so that less than 0.05 ppm basic nitrogen remains in the feed to the oligomerization reactor. Preferably hydrogen is present, in a roughly 0.01:1 to 0.5:1 molar ratio with hydrocarbon feed. Use of a two stage feed pretreatment process, with an aqueous primary stage and zeolitic secondary stage, will provide enhanced operational stability.

Although fixed bed operation of the oligomerization reactor is preferred, because of simplicity and low cost, it is also possible to operate with a fluidized bed or with a moving bed design, which permits continuous removal and replacement of spent catalyst. Swing reactors are another way to tolerate a higher catalyst aging rate while remaining on stream all the time.

Although the practice of the present invention greatly extends catalyst cycle lengths, there will be a gradual accumulation of coke and gum material which will cause deactivation of the oligomerization catalyst. Hot hydrogen stripping will, in many instances, restore some catalyst activity. Conventional hydrogen stripping conditions may be used. For complete regeneration of the catalyst contact with an oxygen containing gas, preferably air added to a circulating nitrogen stream, can be used to burn off coke and gummy hydrocarbon deposits. Conventional catalyst regeneration conditions can be used.

The process of the present invention provides refiners with an efficient way of upgrading light, relatively low value, olefinic streams to more valuable gasoline and distillate products. The process is especially efficient at converting C₂ to C₅ olefins into more valuable products, despite the presence of relatively large amounts of basic nitrogen in such refinery streams. The process is very tolerant of light olefins such as ethylene and can easily accommodate heavy olefins boiling in the naphtha range. With reduced limits on gasoline RVP (Reid Vapor Pressure) refiners now have a way to increase gasoline yield without adding more light ends to the gasoline pool. Light olefin streams can be upgraded into more valuable, heavier liquid products without resort to HF or sulfuric acid alkylation.

## Claims

1. A process for upgrading a C₂-C₁₀ olefinic feed containing basic nitrogen compounds, produced by thermal or catalytic cracking of a heavier liquid hydrocarbon and selected from coker naphtha, visbreaker naphtha, pyrolysis naphtha, olefinic naphtha from cracking, refinery-produced LPG streams comprising 2-5 carbon atom hydrocarbons and normally gaseous 3-4 carbon atom hydrocarbon streams produced by cracking, said process comprising contacting the olefinic feed with a catalyst comprising a zeolite having a Constraint Index of 1 to 12 and a silica to alumina mole ratio of at least 12 at superatmospheric pressure and in the presence of hydrogen at a hydrogen to hydrocarbon ratio of about 0.01:1 to 1:1 to oligomerize at least a portion of the olefins in the feed to gasoline and distillate boiling range products.

2. A process according to claim 1 wherein the olefinic feed contains more than 1 wt ppm of basic nitrogen compounds, and the basic nitrogen content of the feed is reduced by a feed pretreatment step prior to upgrading to reduce the basic nitrogen content of the feed below 0.1 wt ppm basic nitrogen.

3. A process according to claim 2 wherein the basic nitrogen content of the feed is reduced below 0.05 wt ppm by pretreatment.

4. A process according to claim 2 or claim 3 wherein the feed pretreatment step comprises water washing or contact with a solid adsorbent having an affinity for basic nitrogen compounds.

5. A process according to claim 4 wherein the pretreated feed is further pretreated by contact with a bed of large pore zeolite or of a zeolite having a Constraint Index of 1 to 12.

6. A process according to any preceding claim wherein the hydrogen to hydrocarbon ratio is 0.05 to 0.5.

7. A process according to any preceding claim wherein the oligomerization reaction conditions include a pressure of 7.9 to 70 bar (100 to 1000 psig), a temperature of 121 to 399°C (250 to 750°F) and a hydrogen:hydrocarbon mole ratio of 0.2:1 to 1:1.

8. A process according to any of claims 1 to 7 wherein the feed contains more than 0.5 wt ppm basic nitrogen compounds and comprises olefinic hydrocarbons having three to four carbon atoms which constitute the lighter products obtained by catalytic cracking of heavy, sulfur containing hydrocarbons boiling in the gas oil and heavier range to lighter products and H₂S, said lighter products having been treated with an amine to remove H₂S.

9. A process according to claim 8 wherein the oligomerization catalyst comprises ZSM-5 and the basic nitrogen content of said feed is reduced below 0.05 wt ppm by pretreatment before contact with the oligomerization catalyst.

10. A process according to any preceding claim wherein the oligomerization is conducted at a hydrocarbon feed weight hourly space velocity of 0.2 to 20.

11. A process according to any preceding claim wherein said catalyst comprises a zeolite having an alpha activity greater than 100.

12. A process according to any preceding claim wherein the oligomerization is conducted at a temperature; of 149 to 288°C (300-550°F) and a hydrocarbon weight hourly space velocity of 0.5 to 5, and with a zeolite having an alpha activity above 150.

13. A process according to any preceding claim wherein the feed is an unstable naphtha comprising dienes and olefins produced by thermal cracking of a heavy hydrocarbon charge.

14. A process according to claim 13 wherein said thermal cracking is visbreaking or coking.

15. A process according to any of claims 4 to 14 wherein said solid adsorbent comprises silica gel, an ion exchange resin or alumina.

16. A process according to claim 15 wherein said alumina is activated alumina.

17. A process according to any of claims 13 to 16 wherein the feed contains more than 0.25 wt% dienes.

18. A process according to any of claims 13 to 17 wherein the oligomerization is carried out at a temperature of 107 to 260°C, a pressure of 7.9 to 70 bar and a naphtha weight hourly space velocity of 0.2 to 20.

19. A process according to claim 18 wherein the oligomerization is carried out at a temperature of 121 to 232°C, a pressure of 21.7 to 35.5 bar and a weight hourly space velocity of 0.5 to 5.

## Patentansprüche

1. Verfahren zur Veredlung einer C₂-C₁₀-Olefinbeschickung, die basische Stickstoffverbindungen enthält, die durch thermisches oder katalytisches Cracken eines schwereren flüssigen Kohlenwasserstoffs erzeugt wurde und aus Rohbenzin der Coking-Anlage, Rohbenzin der Visbreaking-Anlage, Rohbenzin von der Pyrolyse, olefinischem Rohbenzin vom Cracken, in der Raffinerie erzeugten LPG-Strömen, die Kohlenwasserstoffe mit 2 bis 5 Kohlenstoffatomen umfassen, und normalen gasförmigen Kohlenwasserstoffströmen mit 3 bis 4 Kohlenstoffatomen, die durch Cracken erzeugt wurden, ausgewählt ist, wobei dieses Verfahren den Kontakt der olefinischen Beschickung mit einem Katalysator, der einen Zeolith mit einem Zwangsindex von 1 bis 12 und einem Siliciumdioxid/Aluminiumoxid-Molverhältnis von mindestens 12 umfaßt, bei Überdruck und in Gegenwart von Wasserstoff bei einem Wasserstoff/Kohlenwasserstoff-Verhältnis von etwa 0,01:1 bis 1:1 umfaßt, wodurch mindestens ein Teil der Olefine in der Beschickung in Produkte im Siedebereich von Benzin und Destillat umgewandelt wird.

2. Verfahren nach Anspruch 1, wobei die Olefinbeschickung mehr als 1 Gew.-Teile pro Million basische Stickstoffverbindungen enthält und der Gehalt der Beschickung an basischem Stickstoff vor der Veredlung durch einen Vorbehandlungsschritt der Beschickung verringert wird, wodurch der Gehalt der Beschickung an basischem Stickstoff auf weniger als 0,1 Gew.-Teile pro Million basischen Stickstoff verringert wird.

3. Verfahren nach Anspruch 2, wobei der Gehalt der Beschickung an basischem Stickstoff durch die Vorbehandlung auf weniger als 0,05 Gew.-Teile pro Million verringert wird.

4. Verfahren nach Anspruch 2 oder 3, wobei der Schritt der Vorbehandlung der Beschickung eine Wasserwäsche oder den Kontakt mit einem festen Adsorptionsmittel umfaßt, das eine Affinität für basische Stickstoffverbindungen aufweist.

5. Verfahren nach Anspruch 4, wobei die vorbehandelte Beschickung außerdem durch den Kontakt mit einem Bett eines großporigen Zeolith oder eines Zeolith mit einem Zwangsindex von 1 bis 12 vorbehandelt wird.

6. Verfahren nach einem der vorstehenden Ansprüche, wobei das Verhältnis von Wasserstoff zu Kohlenwasserstoff 0,05 bis 0,5 beträgt.

7. Verfahren nach einem der vorstehenden Ansprüche, wobei die Bedingungen der Oligomerisierungsreaktion einen Druck von 7,9 bis 70 bar (100 bis 1000 psig), eine Temperatur von 121 bis 399°C (250 bis 750°F) und ein Molverhältnis von Wasserstoff:Kohlenwasserstoff von 0,2:1 bis 1:1 umfassen.

8. Verfahren nach einem der Ansprüche 1 bis 7, wobei die Beschickung mehr als 0,5 Gew.-Teile pro Million basische Stickstoffverbindungen enthält und olefinische Kohlenwasserstoffe mit 3 bis 4 Kohlenstoffatome umfaßt, die die leichteren Produkte darstellen, die durch das katalytische Cracken von hochsiedenden, schwefelhaltigen Kohlenwasserstoffen, die im Bereich von Gasöl und darüber sieden, zu leichteren Produkten und H₂S erhalten wurden, wobei die leichteren Produkte mit Amin behandelt wurden, wodurch H₂S entfernt wurde.

9. Verfahren nach Anspruch 8, wobei der Oligomerisierungskatalysator ZSM-5 umfaßt und der Gehalt der Beschickung an basischem Stickstoff vor dem Kontakt mit dem Oligomerisierungskatalysator durch Vorbehandlung auf weniger als 0,05 Gew.-Teile pro Million verringert wird.

10. Verfahren nach einem der vorstehenden Ansprüche, wobei die Oligomerisierung bei einer stündlichen Gewichts-Raum-Geschwindigkeit der Kohlenwasserstoffbeschickung von 0,2 bis 20 durchgeführt wird.

11. Verfahren nach einem der vorstehenden Ansprüche, wobei der Katalysator einen Zeolith mit einer α-Aktivität von mehr als 100 umfaßt.

12. Verfahren nach einem der vorstehenden Ansprüche, wobei die Oligomerisierung bei einer Temperatur von 149 bis 288°C (300 - 550°F) und einer stündlichen Gewichts-Raum-Geschwindigkeit der Kohlenwasserstoffe von 0,5 bis 5 und mit einem Zeolith mit einer α-Aktivität von mehr 150 durchgeführt wird.

13. Verfahren nach einem der vorstehenden Ansprüche, wobei die Beschickung ein instabiles Rohbenzin ist, das Diene und Olefine umfaßt und durch thermisches Cracken einer hochsiedenden Kohlenwasserstoffbeschickung erzeugt wurde.

14. Verfahren nach Anspruch 13, wobei das thermische Cracken das Visbreaking oder Coking ist.

15. Verfahren nach einem der Ansprüche 4 bis 14, wobei das feste Adsorptionsmittel Kieselgel, ein Ionenaustauschharz oder Aluminiumoxid umfaßt.

16. Verfahren nach Anspruch 15, wobei das Aluminiumoxid aktiviertes Aluminiumoxid ist.

17. Verfahren nach einem der Ansprüche 13 bis 16, wobei die Beschickung mehr als 0,25 Gew.-% Diene enthält.

18. Verfahren nach einem der Ansprüche 13 bis 17, wobei die Oligomerisierung bei einer Temperatur von 107 bis 260°C, einem Druck von 7,9 bis 70 bar und einer stündlichen Gewichts-Raum-Geschwindigkeit des Rohbenzins von 0,2 bis 20 durchgeführt wird.

19. Verfahren nach Anspruch 18, wobei die Oligomerisierung bei einer Temperatur von 121 bis 232°C, einem Druck von 21,7 bis 35,5 bar und einer stündlichen Gewichts-Raum-Geschwindigkeit von 0,5 bis 5 durchgeführt wird.

## Revendications

1. Un procédé de valorisation d'une charge d'oléfines en C₂ à C₁₀ contenant des dérivés basiques de l'azote, obtenue par craquage catalytique ou thermique d'un hydrocarbure liquide plus lourd choisi parmi naphta d'unités de cokéfaction, naphta issu d'une opération de réduction de la viscosité, naphta de pyrolyse, naphta oléfinique provenant de craquage, courants de LPG obtenus en raffinerie comprenant des hydrocarbures à 2 à 5 atomes de carbone et courants d'hydrocarbures renfermant de 3 à 4 atomes de carbone normalement gazeux obtenus par craquage, ce procédé comprenant la mise en contact d'une charge oléfinique avec un catalyseur comprenant une zéolite dont l'indice de contrainte est compris entre 1 et 12 et le rapport molaire silice/alumine est au moins égal à 12, à une pression supérieure à la pression atmosphérique et en présence d'hydrogène avec un rapport hydrogène/hydrocarbures compris entre environ 0,1/1 et 1/1 pour oligomériser au moins une partie des oléfines dans la charge en produits bouillant dans l'intervalle des essences et des distillats.

2. Un procédé selon la revendication 1, dans lequel la charge oléfinique contient plus de 1 ppm en poids de dérivés basiques de l'azote et la teneur en azote basique de la charge est réduite par une étape de prétraitement de la charge avant la valorisation pour réduire la teneur en azote basique de la charge à une valeur inférieure à 0,1 ppm en poids d'azote basique.

3. Un procédé selon la revendication 2, dans lequel la teneur en azote basique de la charge est réduite à une valeur inférieure à 0,05 ppm en poids par prétraitement.

4. Un procédé selon la revendication 2 ou 3, dans lequel l'étape de prétraitement de la charge comprend un lavage à l'eau ou la mise au contact avec un adsorbant solide présentant une affinité pour les dérivés basiques de l'azote.

5. Un procédé selon la revendication 4, dans lequel la charge prétraitée subit un autre prétraitement par mise en contact avec un lit constitué d'une zéolite à larges pores ou d'une zéolite dont l'indice de contrainte est compris entre 1 et 12.

6. Un procédé selon l'une quelconque des revendications précédentes, dans lequel le rapport hydrogène/hydrocarbures est compris entre 0,05 et 0,5.

7. Un procédé selon l'une quelconque des revendications précédentes, dans lequel les conditions de la réaction d'oligomérisation comprennent une pression de 7,9 à 70 bar (100 à 1000 psig), une température de 121 à 399°C (250 à 750°F) et un rapport molaire hydrogène/hydrocarbures compris entre 0,2/1 et 1/1.

8. Un procédé selon l'une quelconque des revendications précédentes, dans lequel la charge contient plus de 0,5 ppm en poids de dérivés basiques de l'azote et comprend des hydrocarbures oléfiniques renfermant de 3 à 4 atomes de carbone qui constituent les produits légers obtenus par craquage catalytique d'hydrocarbures lourds contenant du soufre, bouillant dans l'intervalle des gazoles et des produits plus lourds, en produits plus légers et H₂S, lesdits produits plus légers ayant été traités par une amine pour éliminer H₂S.

9. Un procédé selon la revendication 8, dans lequel le catalyseur d'oligomérisation comprend de la ZSM-5 et la teneur en azote basique de cette charge est réduite en-dessous de 0,05 ppm en poids par prétraitement avant contact avec le catalyseur d'oligomérisation.

10. Un procédé selon l'une quelconque des revendications précédentes, dans lequel l'oligomérisation est conduite avec une vitesse spatiale horaire pondérale de la charge d'hydrocarbures comprise entre 0,2 et 20.

11. Un procédé selon l'une quelconque des revendications précédentes, dans lequel ce catalyseur comprend une zéolite dont l'activité alpha est supérieure à 5.

12. Un procédé selon l'une quelconque des revendications précédentes, dans lequel l'oligomérisation est conduite à une température de 149 à 288°C (300 à 550°F) et avec une vitesse spatiale horaire pondérale d'hydrocarbures de 0,5 à 5 et en présence d'une zéolite dont l'activité alpha est supérieure à 150.

13. Un procédé selon l'une quelconque des revendications précédentes, dans lequel la charge est un naphta instable, comprenant des diènes et des oléfines, obtenu par craquage catalytique d'une charge d'hydrocarbures lourds.

14. Un procédé selon la revendication 13, dans lequel ledit craquage catalytique est une opération de réduction de la viscosité ou une cokéfaction.

15. Un procédé selon l'une quelconque des revendications 4 à 14, dans lequel ledit adsorbant solide comprend du gel de silice, une résine échangeuse d'ions ou de l'alumine.

16. Un procédé selon la revendication 15, dans lequel ladite alumine est une alumine activée.

17. Un procédé selon l'une quelconque des revendications 13 à 16, dans lequel la charge contient plus de 0,25% en poids de diènes.

18. Un procédé selon l'une quelconque des revendications 13 à 17, dans lequel l'oligomérisation est mise en oeuvre à une température de 107 à 260°C, une pression de 7,9 à 70 bar et une vitesse spatiale horaire pondérale de naphta de 0,2 à 20.

19. Un procédé selon la revendication 18, dans lequel l'oligomérisation est mise en oeuvre à une température de 121 à 232°C, une pression de 21,7 à 35,5 bar et une vitesse spatiale horaire pondérale de 0,5 à 5.
